# EUROPEAN PATENT APPLICATION

(11) **EP 1 493 416 A2**
(43) Date of publication of application: **05.01.2005**
(21) Application number: 04254009.6
(22) Date of filing: 02.07.2004
(51) Int. Cl.: A61F 13/511

(54) **Flexible form fitting web**

(30) Priority: 02.07.2003 US 484432 P
(71) Applicant: Tredegar Film Products Corporation, Richmond, VA 23225 (US)
(72) Inventor: Thomas, Paul Eugene, Terre Haute IN 47802-5066 (US); Chung, Tze Wan Pansy, Fox River Grove IL 60021 (US)
(74) Representative: Hayes, Adrian Chetwynd

(57) **Abstract**

A flexible form fitting web comprises a repeating pattern of predominant high stress lanes normal to the stretch force axis, which lanes are defined by depressions on both sides of the lanes. Connecting members transmit the stretch force to the high stress lanes causing the lanes to respond to stress by bending and uncoupling in varying degrees of each.

## Description

### TECHNICAL FIELD

A polymeric web used in garments, undergarments, disposable garments and absorbent articles inserted into garments, and more particularly where comfort is desired via a form fitting of the web in intimate contact with the body during activities causing bodily motions.

### BACKGROUND

As the world industrializes and the income base of people continues to rise, an increase in time spent in recreational athletic activity is also increasing. Exercising to stay in shape is also becoming increasingly popular with the increase in sedentary work in this age of information. These athletic activities put unusual stress on garments being worn at the time of the activity, most commonly a stretch force whereupon certain segments of the garment are repeatedly stretched and relaxed during the bodily motions associated with the activity. The stretch force is generally aligned in a single axis and the axis is usually aligned in the axis corresponding to the body motion. A common area of concern is in the crotch region. This is especially true concerning women who want to maintain comfort and protection while being active and who may also insert an absorbent article into their undergarments.

A stretch force can be applied to the undergarment, and to an absorbent article inserted into the undergarment, by motions associated with this region. Such motions may include lifting the legs, kicking, splitting the legs laterally, sidestepping or sideways shuffling, or crossing and uncrossing one's legs while sitting. Rotating a leg will also cause a moment of lateral stretch force, and common motions such as walking or running straight ahead, where the legs stroke forward and backward, but do not considerably separate, may create a consequential stretch force depending on the comparative anatomy of various individuals. It is also understood that when a stretch force is applied it is typically relaxed at a later time.

When a stretch force is applied to materials that lack adequate flexibility, such stretch forces can cause rupture, displacement, wrinkling, crumpling, bunching and other distortions that are uncomfortable to the wearer. Comfort during these situations is best achieved by a flexibility that provides a form fitting function. Form fitting basically means that the web fits to the form of the anatomy with which it is in contact. As the body moves and applies stress to both the anatomy and the web, both must undergo a similar shape change. The human anatomy is highly flexible and can easily change shape in response to the stretch force being applied and relaxed. Few webs are known which have the flexibility to move with the body and undergo the similar shape shifts while remaining in intimate contact with the body in order to yield the desired comfort factor.

One web, sold under the trademark "BARELY THERE," a registered trademark of The Sara Lee Corporation, has threads made of microfibers, which are knitted into special interconnected loop patterns. The flexibility of microfibers, which are fibers with a diameter of less than 10µm, combined with the looped knitting patterns provides a unique form fitting flexibility. The "BARELY THERE" trademark is used on a line of panty undergarments. Women questioned in market focus groups have indicated that these panties are light, comfortable and form fitting. The panty's ability to conform to the anatomy, as well as remain form fitting during various bodily motions that cause a stretch force, yields a desired comfort factor. While this type of web is suitable for panties, it does not have desirable strikethrough and rewet characteristics and is therefore not suitable as the body side layer for absorbent articles such as disposable panty liners which are designed to be inserted into the panties.

Other webs, however, have suitable strikethrough and rewet characteristics to act as the body side layer for these types of insertable absorbent articles. These webs, however, generally lack the form fitting flexibility. Disposable absorbent article webs are typically nonwoven webs made of randomly laid and bonded polymeric fibers. An alternative to these nonwoven webs is a cover material having a formed polymeric web with a pattern of fiber-like elements and a specialized microtexture, for example that sold under the trademark "DRI-WEAVE," a registered trademark of The Procter & Gamble Company. These types of webs are most commonly made of non-elastomeric polymers. It is known in the art, however, that these types of web layers lack the desired flexibility required for a satisfactory form fitting function.

Attempts have been made to incorporate elastomeric polymers into various types of disposable webs used for insertable absorbent articles. Elastomeric polymers are easily added to the polymeric formed webs such as DRI-WEAVE™ by blending various polymers together in a dry blend of pellets. Some nonwovens have also been made from elastomeric polymers, but this is more difficult since a fiber must draw down to a very small diameter and elastomerics, by nature, tend to spring back versus draw down. While some of these elastomeric formed webs have achieved significant stretch and recovery values, they are generally not form fitting and have lacked the aesthetic and tactile attributes typically required for direct body contact applications. Elastomeric materials tend to look and feel rubbery as opposed to more desirable aesthetic and tactile impressions, such as cottony or silky. Elastomeric webs are therefore often limited to uses such as fastening strips and the like.

### SUMMARY

The embodiments described herein are drawn to a material, an article including the material, and a method of making a material that is form fitting.

In one embodiment, the material is a flexible web that is form fitting to the anatomy when in intimate contact with the body during activities with various bodily motions that cause a stretch force axis affecting the web. The web can be made entirely from non-elastomeric polymers yet it exhibits flexibility parameters similar to webs made with elastomeric polymers. The web has a repeating pattern of predominant high stress lanes normal to the stretch force axis defined by depressions on both sides of the lanes. The lanes are interconnected to the stretch force axis by connecting members, which interrupt the depressions. These connecting members transmit the stretch force to the high stress lanes. The high stress lanes react to the stretch force by bending and uncoupling to varying degrees and can therefore respond to both the application of the stretch force and the relaxation of the stretch force. The web has a specific pattern of lanes with set relationships of lane width, lane spacing, depression depth, and, in some embodiments, connecting member placement. The web has a relationship of its 5% Stress value in the stretch force axis being no more than 30% of its 5% Stress value in the high stress lane axis.

In another embodiment a web includes a repeating pattern of elongated high stress lanes aligned substantially normal to a stretch force axis. The pattern of high stress lanes is defined by a plurality of depressions which protrude away from an uppermost contacting plane of the web. The depressions having substantially vertical sidewalls. The high stress lanes have a width defined by a distance between the substantially vertical sidewalls of the depressions on either side of the lane. The repeating pattern of lanes have a separation distance between the lanes. The depressions are interrupted by connecting members extending between adjacent high stress lanes.

A 5% Stress value for the stretch force axis may, in one instance, be no more than about 30% of a 5% Stress value in the high stress lane axis. The lane width may, in one instance, be at least about 300µm to at most about 1,500µm. The depression depth may, in one instance, be from about 100% the lane width to about 300% the lane width. The lanes may repeat with the separation distance from about 100% of the lane width to about 200% of the lane width. The connecting members may, in one instance, be in a lower plane than an uppermost plane of the high stress lanes. The high stress lanes may exhibit twisted thread lines. The connecting members may, in one instance, be essentially coplanar with an uppermost plane of the high stress lanes. The depressions may have substantially pointed ends for unhinging during bending. The connecting members may interrupt the depressions about once every 1,250µm to about once every 12,500µm. The web may, in one instance, be made from a blend comprising no elastomeric polymers. The depressions may, in one instance, be apertures. Patterned zones of apertured and unapertured depressions may coexist.

In another embodiment a flexible form fitting web includes a plurality of elongated depressions having substantially vertical sidewalls along a length of the depressions and connecting members at ends of the depressions. A plurality of elongated high stress lanes are between the depressions. The high stress lanes extend in a machine direction and are connected to one another by the connecting members. They are separated from one another by the depressions. The high stress lanes and the connecting members each have an upper surface substantially in the same plane.

A 5% Stress value for a stress force axis may, in one instance, be no more than about 30% of the 5% Stress value in a high stress lane axis. A lane width may, in one instance, be at least about 10µm to at most about 5,000µm. A depression depth may, in one instance, be at least about 25% the lane width to at most about 500% the lane width. The lanes may repeat with a separation distance of at least about 2% of the lane width to at most about 400% of the lane width. The depressions may, in one instance, be apertures.

In another embodiment, an article for the absorption of bodily fluids includes a flexible absorbent core, a flexible barrier layer, and a flexible body contacting layer with a repeating pattern of predominant high stress lanes aligned normal to a stretch force axis. The pattern of high stress lanes are defined by depressions which protrude away from the uppermost contacting plane of the web. A lane width is effectively defined by a distance between a substantially vertical component of the depressions on both sides of the lane. The repeating pattern of lanes are defined by a separation distance between the lanes. The depressions are interrupted by connecting members. The connecting members transmit the stretch force to the high stress lanes by being affixed to sides of the high stress lanes.

The details of the embodiments are set forth in the accompanying drawings and the description below. Other features, objects, and advantages of the embodiments will be apparent from the description and drawings, and from the claims.

### DESCRIPTION OF DRAWINGS

FIG. 1 is a plan view of the body contacting surface of "BARELY THERE™" web found in the crotch region of a panty undergarment.

FIG. 2 is a plan view of the body contacting surface of DRI-WEAVE™ web found as a cover layer on several absorbent articles insertable into a panty undergarment.

FIG. 3 is a plan view of the body contacting surface of a web according to an embodiment.

FIG. 4 is a cross-sectional view of the web of FIG. 3 taken across several high stress lanes.

FIG. 5 is a sketch of the cross-section of the web of FIG. 3 taken across several high stress lanes and connecting members.

FIG. 6 is a plan view of the body contacting surface of a web of an embodiment having a repeating pattern of predominant high stress lanes that resemble thread like elements placed on a coarse mesh pattern.

FIG. 7 is plan view of the body contacting surface of a web according to an embodiment of the invention having a repeating pattern of predominant high stress lanes that resemble thread like elements placed on a fine mesh pattern.

FIG. 8 is a cross-sectional view of the web of FIG. 7 taken across several high stress lanes and connecting members.

FIG. 9 is a plan view of the body contacting surface of a web of an embodiment having a repeating pattern of predominant high stress lanes that resemble a special weave pattern.

Like reference symbols in the various drawings indicate like elements.

### DETAILED DESCRIPTION

### Definitions

The term "stretch force axis" means the general axis of the stretching or elongating stress applied to the web as caused by various bodily motions associated with bodily activities. The stretch force axis will exist substantially in one axis. The stretch force axis will most often be aligned in the same direction as the motion itself. For example, in the crotch region of undergarments, the stretch force axis may be aligned laterally or approximately 90° to the "straight ahead" direction of the human subject, which is defined as the direction they are facing. While this is the most typical stretch force direction, it is to be understood that this may not be the only one.

The phrase "predominant high stress lanes" as used herein describes a repeating pattern of lanes of material that exhibit a substantially higher value of stress when strained in their common axis than when the web is strained in the stretch force axis. The axis of these lanes is substantially normal to the stretch force axis. The predominant high stress lanes provide stability in the web in their common axis, which helps the web maintain its proper position with respect to its placement in the absorbent garment as well as helping to avoid crumpling or creeping in this axis. When the predominant high stress lanes coincide with the machine direction ("MD") of the web, the conversion or machining steps utilized to attach these materials onto their finished composite structures are facilitated by the web's reduced stretching in the MD. This also serves to protect the web from any distortion due to the MD stresses of the conversion processes. As will be discussed in more detail below, the high stress lanes of the illustrative webs described herein define inverted "U" shapes in cross section.

A "lane" represents a predominant pattern of substantially undisturbed material aligned in a common direction. A lane has a width and a length. In most embodiments, the lane's length is continuous, running end to end for the full length of the object. In some embodiments, however, it may be interrupted provided that the interruptions do not diminish the 'predominance' of the lane's pattern. A lane's width is defined by depressions formed on either side of the lane.

The "depressions" are protuberances protruding away from the uppermost plane of the high stress lanes. The depression has a substantially vertical component as it progresses away from the uppermost plane and will eventually terminate. The "depth" of the depression is defined as the Z direction distance from the uppermost plane of the high stress lanes down to the point where the depression terminates. The depressions are interrupted by connecting members. The depressions may terminate as apertures, but they may also terminate as unapertured depressions.

The term "repeating pattern" designates a pattern of lanes such that the lane repeats itself in the stretch force axis. The repeat of the lane is defined by the distance of space between the lanes and the width of the lane. Varying the frequency of the repeat of lanes can affect flexibility. Other factors such as tactile and visual aesthetics may also influence the choice of pattern. The repeating lanes are interconnected by connecting members, which transmit the stretch force to the high stress lanes.

"Connecting members" is a term used herein to describe the material that interconnects the predominant high stress lanes. The connecting members interrupt the depressions with a predetermined frequency. These connecting members transmit the stretch force to the predominant high stress lanes causing the lanes to bend and uncouple in order to absorb the stretch force applied in the stretch force axis. They are connected to the sides of the high stress lanes and may exist in the same plane as the uppermost plane of the lanes or in a lower plane.

The term "uncoupling" is used herein to describe the instance when the inverted "U" cross-sectional shape of the high stress lanes opens up at its base with the application of the stretch force. Its natural, unstressed state is to have two substantially vertical sides in parallel. When the stretch force is applied, the base area opens up becoming non-parallel, or is 'uncoupling'. The cross section will tend to return to its original parallel form.

The term "bending" relates to its common meaning. The middle of the high stress lanes is substantially straight and parallel to other lanes while in a relaxed state, but when a stretch force is applied by the connecting members the lanes will bend in the direction of the stress. The lanes will tend to return to their original parallel form once the stretch force is relieved.

The phrase "5% Stress relationship" defines a relationship between the 5% Stress value, or force required to stretch the material to 105% of its original size, of the web when pulled in the stretch force axis as a percentage derived when that value is divided by the 5% Stress value of the web when pulled in the axis of the predominant high stress lanes. Therefore, a material with a 5% Stress relationship of 20% is a material in which the force required to stretch the material to 105% of its original size in the force axis is 20% of the force required to stretch the material to 105% of its original size in the axis of the predominant high stress lanes.

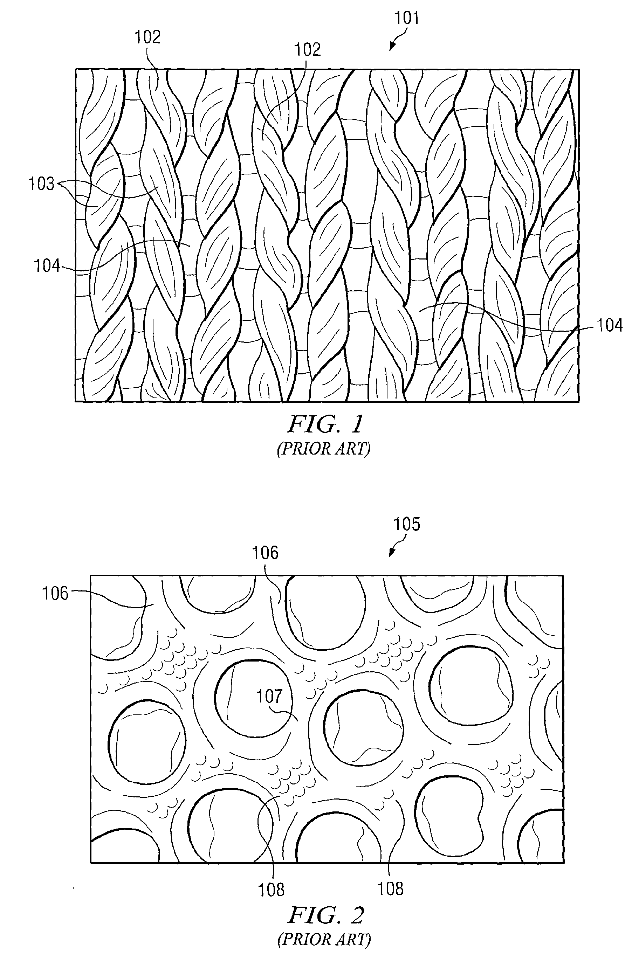

FIG. 1 depicts a plan view of a segment of the "BARELY THERE™" fabric, or material 101. The material 101 is made of microfibers 102, which form threads 103. The threads 103 are knitted into loops 104. The microfibers 102 are 10µm in diameter, or less, so that threads 103 yield and can bend easily when loops 104 are stressed. This makes the material 101 flexible and form fitting. The expense and lack of fluid management functionality of the material 101 makes it unsuitable for disposable items or items intended to be inserted into garments for the purpose of absorbing bodily fluid exudates.

FIG. 2 depicts a plan view of a segment of "DRI-WEAVE™" fabric 105. The DRI-WEAVE™ 105 is very well suited for a body contacting layer intended for absorbent articles of the type inserted into garments for the purpose of absorbing bodily fluid exudates. Fiber-like elements 106 are interconnected to form pentagons 107 which are nested in a repeating pattern. The visual and skin contacting surface of the fabric 105 has a microtexture of microdots 108 to lower the gloss. The combination of fiber-like elements 106 and microdots 108 give fabric 105 a cloth-like look and feel. The nested pentagon pattern, however, can only unfold slightly, at best, when a stretch force is experienced and therefore this fabric does not have the flexibility required to be form fitting.

Turning now to the embodiments described herein, the form fitting flexibility of a web according to the embodiments is derived from the web having a certain repeating pattern of predominant high stress lanes aligned normal to the stretch force axis. The lanes are interconnected to the stretch force axis by connecting members of a specific placement such that the stretch force is applied to the high stress lanes to cause a bending and uncoupling moment in the lanes. Bending and uncoupling may occur at varying degrees. The lanes are specifically designed to avoid reaching their deformation limit in their own longitudinal axis under normal stretch forces, yet the lanes can bend and uncouple when the connecting members transmit the stretch force to them at their connecting points.

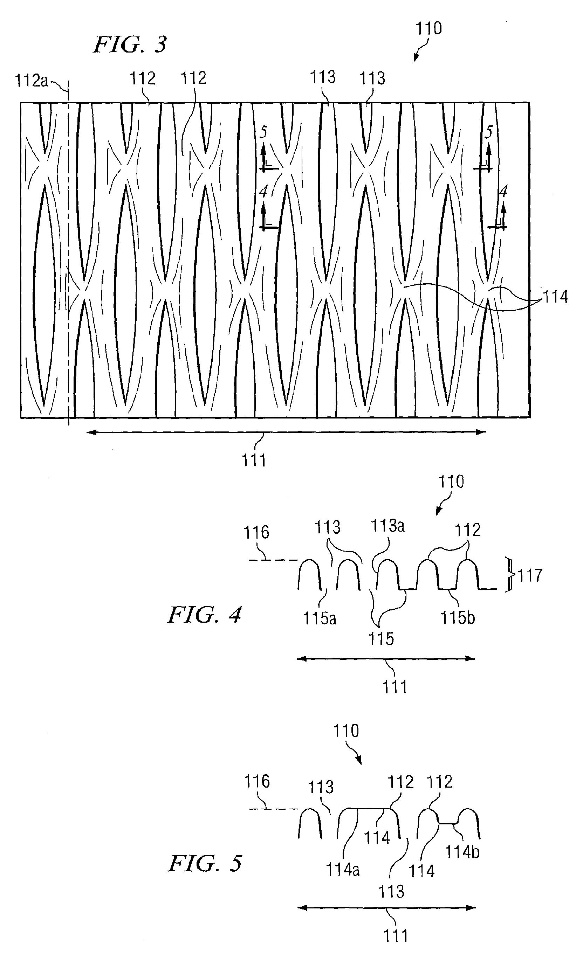

Referring now to FIG. 3 of the drawings, that figure depicts the design parameters of flexible form fitting web 110 as seen in plan view of the body contacting surface. Web 110, which may be a fabric, has a stretch force axis 111 and predominant high stress lanes 112 with a single lane median being depicted by a dash-line 112a. The predominant high stress lanes 112, which are continuous lanes, are normal to stretch force axis 111. Depressions 113 exist on both sides of lanes 112 and define the repeating pattern of lanes 112. Connecting members 114 interrupt depressions 113 and connect the sides of adjacent lanes 112 and transmit stretch force between the lanes 112.

Referring now to FIG. 4 of the drawings, the cross-sectional view depicted cuts across five high stress lanes 112. Web 110 has a stretch force axis 111 and high stress lanes 112 defined by depressions 113. Depressions 113 have a substantially vertical component 113a, which define the width of lanes 112. Depressions 113 have a depth 117, which begin in the uppermost plane 116 of web 110 and protrude away from plane 116 until ending at its point of termination 115. Depressions 113 may terminate as apertures 115a or may terminate as unapertured ends 115b.

Referring now to FIG. 5 of the drawings, the cross-sectional view depicts cuts across five high stress lanes 112 and two connecting members 114. Web 110 has a stretch force axis 111 and high stress lanes 112 defined by depressions 113. Connecting members 114 connect to the sides of lanes 112. Connecting member 114a connects to the lanes 112 in a common plane 116. Connecting member 114b connects in a lower plane.

In FIG. 3, the web 110 has high stress lanes 112 defined by depressions 113 which are interrupted by connecting members 114. High stress lanes 112 are aligned normal to stretch force axis 111. Lanes 112 and connecting members 114 are both in the uppermost plane of web 110. In the embodiment of FIG.. 3, the web 110 provides flexibility mostly by bending the high stress lanes 112 when the stretch force is applied in axis 111 by the connecting members 114. The depressions 113 provide a shape having substantially pointed ends, although they need not be as pointed as those depicted in the figure. These substantially pointed ends will hinge during bending. Such hinging enables the web pattern to achieve a low 5% Stress relationship, merely 1% for one embodiment, as shown later.

The particular pattern depicted in FIG. 3 has a lane width of about 130µm and a depression depth of about 160% of the lane's width. In this embodiment the lanes and connecting members both coexist in essentially the uppermost plane. For this pattern, the connecting members interrupt the depressions on a frequency of about one in every 14,000µm. The lanes are spaced apart in a repeating pattern with a separation distance of about 73% of the lane's width. The web is formed on a forming cylinder, which has a similar pattern.

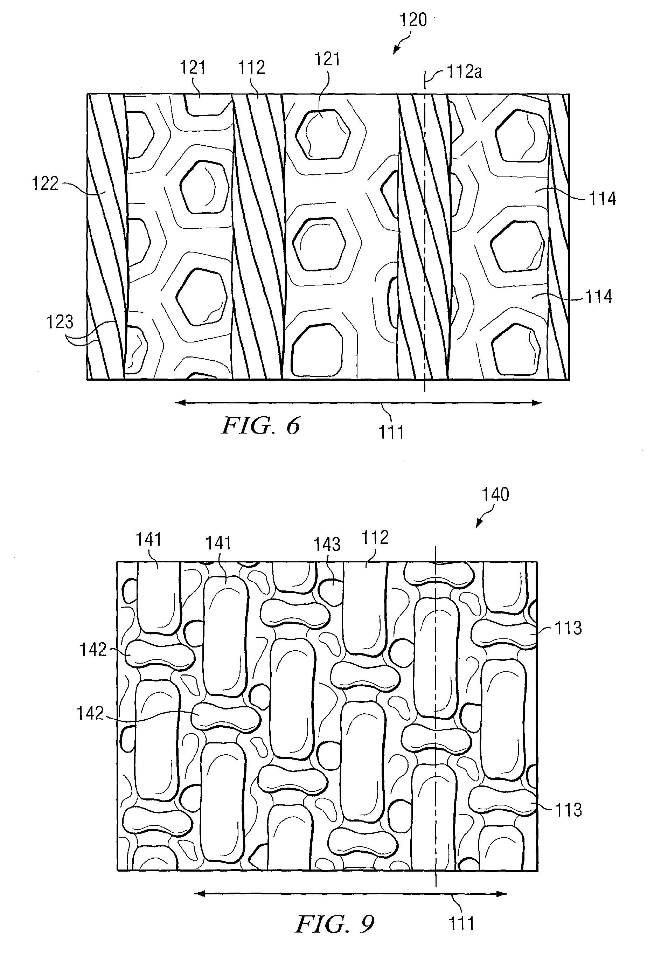

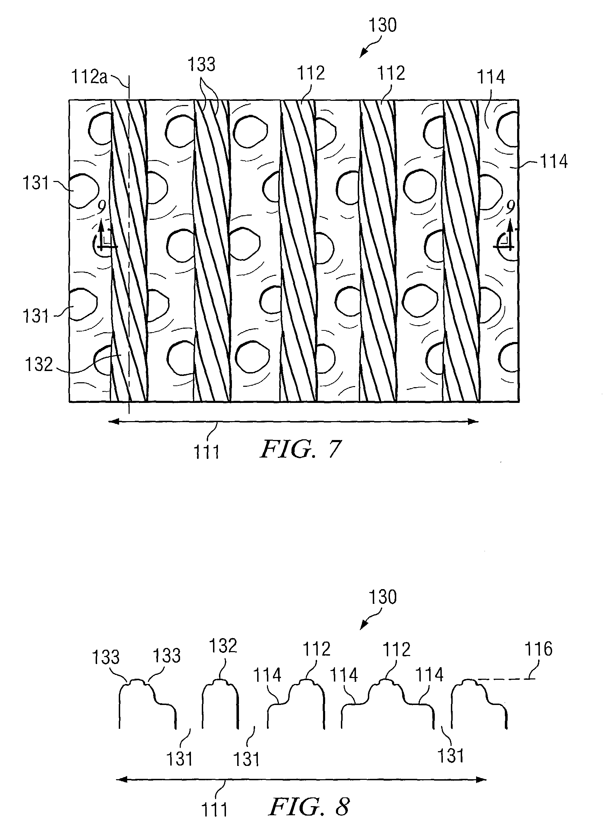

FIG. 6 depicts a segment of web 120 of another embodiment. Web 120 has a base pattern of a mesh pattern formed from a forming cylinder with a corresponding pattern. The pattern is coarse relative to the pattern discussed below with respect to FIG. 7. For example, the forming cylinder can have 22 depressions per linear inch in their aligned direction with the depressions being arrayed in a pattern of equilateral triangles on a 60° array. The mesh pattern defines depressions 121 of web 120. In FIG. 6, the depressions 121 are apertured in order to provide for the transmission of bodily fluid exudates. Depressions 121 define connecting members 114. Elements 122 are provided in the web 120 in an upper plane and exhibit twisted thread lines 123. Elements 122 form high stress lanes 112 which are aligned normal to stress force axis 111. Connecting members 114 are in a lower plane than high stress lanes 112.

Referring now to FIG. 7 of the drawings, this figure depicts a segment of web 130 of another embodiment. FIG. 8 depicts a cross-sectional view of the web of FIG. 7. Web 130 has a base pattern of a mesh pattern formed from a forming cylinder with a corresponding pattern. The pattern is fine relative to the pattern of FIG. 6. For example, the forming cylinder can have 40 depressions per linear inch in their aligned direction with the depressions being arrayed in a pattern of equilateral triangles on a 60° array. The mesh pattern defines depressions 131 of web 130, depressions 131 are depicted as being apertured but some or all may be unapertured. Depressions 131 define connecting members 114. Elements 132 are in an upper plane of the web 130 and exhibit twisted thread lines 133. Elements 132 form high stress lanes 112 which are aligned normal to stress force axis 111. Connecting members 114 are in a lower plane than lanes 112. Note that the cross sections of FIG. 6 and 7 are similar.

The embodiments shown in FIG. 6 and FIG. 7 of the drawings have repeating patterns of high stress lanes in the form of elements 122 and 132 aligned normal to the stretch force axis. The connecting members 114 connect to these high stress lanes 112 in a lower plane. When the connecting members 114 in a lower plane transmit the stretch force to the lanes 112 along axis 111, the lanes 112 will primarily uncouple and will only partially bend. For this reason it is not essential that the connecting members have a specialized pattern or frequency since providing bending capacity in these embodiments is less essential.

In the embodiments of FIGS. 6 and 7, the pattern of connecting members can be designed for other purposes such as aesthetics or fluid management concerns. Depressions 121 and 131 of FIG. 6 and FIG. 7 are of a 22 mesh array and a 40 mesh array pattern, respectively, intended to impart certain aesthetics to the web. Since lanes 112 are above the lower plane of the connecting members 114, a desire that the depression have a depth of at least about 25% of the lane's width is more readily satisfied. The depression depth caused by the difference between the upper and lower planes is additive to the depth of the depressions 121 and 131 as they continue protruding beyond the connecting member plane to their ultimate point of termination. This configuration provides for superior uncoupling with reduced hindrance to any bending that may also be occurring.

The lanes 112 can be formed using a standard forming cylinder that has been modified. Web 130 of FIGS. 6 and 7 were formed from standard patterned cylinder whereupon metal threads, such as single or multifilament wire, were affixed to the outside of the cylinder in a desired pattern. In the embodiment of FIG. 7, a multifilament wire of 460µm diameter comprising a twist of seven smaller wires was used. The metal thread diameter determines the high stress lane width.

The forming cylinder used for FIG. 7 used a perforated cylinder with a pattern of 40 mesh (mesh being perforations per linear inch (2.54cm) in the aligned direction of the perforations), 60° equilateral triangle array pattern of perforations yielding about 285 perforations per square centimeter. Grooves are machined in a spiral pattern in the outer perimeter of the cylinder to a depth of about 45% of the metal threads' diameter. This spiral pattern determines the spacing of the high stress lanes. In this embodiment the lanes are spaced apart by a distance of 700µm. The metal threads are then wrapped into the machined grooves and permanently affixed thereto by overplating, gluing, fusion bonding, oven brazing, or other methods of affixing metal.

The connecting members are formed in the original circumferential plane of the forming cylinder where its perforations exist. The connecting members are somewhat randomized because the spiral pattern of the wire is not necessarily synchronous with the cylinder's perforation pattern. As noted earlier, though, this has no critical bearing on the flexibility of this web but may affect fluid flow management if that is a need in a specific application. If so, then special care is taken to avoid fully occluding the original perforation pattern with the wire affixed over it.

Approximately 55% of the wire's diameter is above the original circumferential plane causing the lanes to form in the higher plane; hence, the desire that the depression, in this case getting its upper portion formed by the difference between these planes, have a depth of at least about 25% of the lane's width is satisfied by this 55% factor which has an upper plane to lower plane measured differential value of 250µm for the embodiment of FIG. 7. The web's depressions, which had formed into the perforations of the forming cylinder, also have a depth of about 350µm. Added together, the total depression depth for the embodiment of FIG. 7 is 600µm compared to the lane width of 460µm (the equivalent of the wire rope's diameter). Hence, the depression depth is 130% of the lane's width.

FIG. 9 depicts a segment of web 140 of another embodiment. Web 140 was formed from a forming cylinder with a weave pattern formed by a weave of large and small diameter wires. The large diameter wires on the cylinder gave rise to the elements 141 in the web and the small diameter wires gave rise to the elements 142 on the web, as seen in FIG. 9. Depressions 143 may terminate as apertures. The web 140 has predominant high stress lanes 112 and connecting members 114 which coexist in part in the uppermost plane. Lanes 112 are aligned normal to stretch force axis 111.

Another embodiment can be formed by manipulating a weave pattern such that the high stress lanes are predominant and aligned normal to the stretch force axis. In such a pattern, the predominant high stress lanes are not continuous but are interrupted by the connecting member portions of the weave pattern. It is also noteworthy for this embodiment that the lanes and connecting members will only partially coexist in the uppermost plane. This is due to the weave pattern weaving the wires over and under each other. Both elements will substantially apex in the common uppermost plane, however, and thus they will essentially coexist in this common plane. Several weave structures can achieve this attribute of the web. For example, a weave can comprise a larger wire passing over two thinner wires to form a pattern of predominant repeating lanes normal to the stretch force axis. These weave patterns can be readily achieved when the pattern cylinder is itself a woven cylinder. These cylinders can be made from metal wires, metal threads or polymer wires or threads. When a polymer wire or thread is used for the pattern cylinder, it must have a higher melting point than the polymer in the web as to avoid melting or distorting under the thermal load of the molding process.

The embodiments can be formed on pattern cylinders where a molten or heat softened web is molded to the pattern cylinder. The energy that causes the formation of the molded depressions may be applied by a rubber roll in a nip or by pneumatic pressure differential, which is typically applied by vacuum. Liquid pressure can also be utilized, most typically hydro pressure. The web formed from these patterns may also need some form of texturizing applied to its uppermost plane in order to provide tactile and visual aesthetics desired for a body contacting web. Varieties of textures are well known in the art and are suitable. Random matter finishes, finely packed patterns of diamonds or pyramids, microridges, or other microscopic textures will suffice.

The apertured embodiments will find many uses where liquid or moisture vapor management is desired. They are also slightly more flexible because the aperture separates the lanes making them more mobile to respond to the stretch force. Unapertured embodiments, however, may find uses where a liquid impervious barrier layer is needed. Patterned zones of apertured and unapertured depressions may also coexist in a common sheet.

While the embodiments described herein provide webs that are flexible and form fitting when made from non-elastomeric polymers, this does not preclude use of elastomeric polymers as a part of the blend of polymers and additives. While careful experimentation can optimize an ideal amount for each of the various embodiments and for other variations such as caliper, in all embodiments of this invention described herein the majority of the blend remains non-elastomeric. Substantial elastic properties can overwhelm the mechanical flexibility of these designs and nullify their desired effect.

Polyethylene can be used as the primary thermoplastic polymer of the embodiments. TiO2 particles can be used for white pigmentation and opacifying.

If fluid management is important, the depressions will be apertured and a surfactant may be incorporated into the polymer blend or applied topically later. Corona treatment may also be applied, with or without surfactant. When a polymer web made from non-elastomeric polymers is designed by these criteria, it will become a flexible form fitting web.

In embodiments where at least a portion of the connecting members coexist in a common plane with the lanes, the lanes will primarily tend to bend when the connecting members transmit the stretch force to the lanes at their connecting points and only partially uncouple. Some uncoupling will occur, however, because the depressions that define the lanes have a depth at least 25% greater than the lane's width.

These webs will have more flexibility if the connecting members are staggered. When the connecting members are in essentially the same plane as the lanes, they can interrupt the depressions at a frequency of no more than one per every 850µm along the lane's axis. This connecting member spacing of at least about 850µm provides room for the bending of the lane. If the connecting members are packed too closely, the lanes will not have room to bend and can not absorb the stretch force being transmitted from the connecting members.

The preferred range of frequency of connecting members in these embodiments is one every 1,000µm to no more than one every 20,000µm; however, the most preferred range is from about one every 1,250µm to one every 12,500µm. If the connecting member spacing goes above one every 20,000µm, then it is approaching a point where the bending length of the lane is too large and beginning to lose its ability to pull back once the stretch force is relaxed. It may also become a grossly large pattern deemed undesirable in body contacting applications. In the embodiments, the pattern of predominant high stress lanes 112 is defined by the depressions 113 existing on both sides of a lane.

The depressions 113 are protuberances, which begin in the uppermost plane 116 of the body contacting side and protrude away from that plane and have a component 113a which is substantially vertical in relationship to the horizontal plane 116 of the body contacting surface of the web. Depressions 113 can have a depth 117 of at least about 25% of the lane width and will terminate at an end 115 which may either be apertured 115a or unapertured 115b. It becomes impractical to have a depression depth 117 that is more than 500% of the lane width. The preferred range of depth 117 is from 50% to 350%, with the most preferred range being from about 100% to 300%.

A repeating pattern of predominant high stress lanes 112 aligned normal to the stretch force axis 111 has lanes of a certain width. The width of the lane 112 is defined by the space between the substantially vertical components 113a of the depressions 113 on opposing sides of the lane 112. Lane widths can be as small as about 10µm but should not exceed about 5,000µm. The preferred width is from about 125µm to about 3,500µm, with the most preferred range being from 300µm to 1,500µm.

The repeating pattern of high stress lanes 112 is specifically set to best serve the flexibility of the web and yield the form fitting comfort. Lanes 112 are aligned substantially normal to the stretch force axis 111 and their pattern repeats in the stretch force axis. A lane is spaced apart from the next lane by a separation distance measured between the edges 113a of adjacent lanes 112. The separation distance between edges 113a of the repeating lanes 112 is at least about 2% of the width of the lane and is at most about 400% of the width of a lane. If the lane spacing exceeds 400% of the width of the lane, the connecting members may themselves become counter-predominant and begin to nullify the flexibility of the web in the stretch force axis. The preferred range of spatial separation is from about 50% to 300%, with the most preferred range being from 100% to 200%. The separation space and the lane width will establish the repeat parameter for the repeating lanes. Any balance between the dominance of the axis of the lanes and the connecting members in the stretch force axis can work against flexibility.

The width of the connecting members is determined by the separation distance between the lanes. However, the length of the connecting members, as they interrupt the depressions, is variable provided they do not supercede the predominance of high stress lanes. One skilled in the art will understand that other requirements such as aesthetics, fluid management, or the overall strength of the web will control this setting.

As a flexible form fitting web the values of stress/strain of the high stress lane axis will have a relationship to those similar values tested in the stretch force axis. This relationship helps to identify these webs as being flexible enough to be form fitting. When this relationship exists, the high stress lanes can properly respond to the stretch force transmitted to them by the connecting members. Once the stretch force is transmitted they will absorb the force by either bending or uncoupling, in varying ratios of both, without deforming such that when the stretch force is relaxed they can pull back to their original form. This action maintains the form fitting aspect of these webs while in intimate contact with the anatomy.

A defining stress/strain relationship of a form fitting web of the embodiments, the 5% Stress value, as tested in the stretch force axis is no more than about 30% of that value when tested in high stress lane axis. In some embodiments it can be as low as 1%, and preferably it is about 10% to 20%. This percentage is derived by dividing the stretch force axis 5% Stress value by the high stress lane axis 5% Stress value. As shown in the table below, the popular prior art polymer web can not provide this relationship, and therefore does not provide a form fitting flexibility.

The DRI-WEAVE™ web shown in FIG. 2 is a non-elastomeric polymer pattern formed web. Its unique pattern of nested pentagons with its interconnecting fiber-like elements and microdot microtexture has been a strong performer in both aesthetics and fluid management performance. At best, though, the only provided mechanism for stretch or flexibility is the unfolding of this pattern during a stretch force. This is less than what can be deemed as flexible or form fitting, which is further demonstrated by its 5% Stress relationship of 42%. The embodiments of FIG. 3, FIG. 6, FIG. 7, and FIG. 9 all exhibit a 5% Stress relationship of less than 30%. The 5% Stress value units in the Table 1 below are in grams per lineal centimeter. Note that while they vary in Stress values due to caliper, polymer density or other factors not specifically outlined herein, their unique and innovative construction will yield the 5% Stress relationship of the flexibility at 30% or less. Generally, though, one skilled in the art will know to use primarily low density polymers and use calipers of less than 75µm. The embodiment of FIG. 7 below had a target caliper of 25µm and the embodiment of FIG. 3 below had a caliper of 50µm, for example. This, at least in part, explains the higher Lane Axis 5% Stress value noticed for the embodiment of FIG. 3.

**Table 1**

| **Material** | **Lane Axis 5% Stress** | **Stretch Axis 5% Stress** | **5% Stress Relationship** |
|---|---|---|---|
| DRI-WEAVE™ | 36 | 15 | 42% |
| FIG. 3 | 554 | 4 | 1% |
| FIG. 6 | 86 | 16 | 19% |
| FIG. 7 | 82 | 9 | 11% |
| FIG. 9 | 170 | 46 | 27% |

The basics of the testing methodology for deriving 5% Stress values are outlined in ASTM D-882. Several commercial units exist which can pull the samples to apply tensile force and capture the data in a computer, which then analyzes and records the data. Instron Corporation is widely known for making these units. In such a unit, one generally affixes a strip of web of a specific width into two jaws or clamps which are spaced apart by a predetermined distance. One jaw remains fixed while the other jaw moves upward. The web strip is stretched, often to the point of rupture. The percent elongation beyond the original preset distance of the jaws is the Strain value and the force exerted to the upper jaw by the web, as sensed by a load cell affixed to that jaw, is recorded as the Stress value. For the values in the Table above the strip was 2.54cm wide (1.0 inch) and the jaw separation was 5.08cm (2 inches). The pull speed was 20 in/min (50.8 cm/min). The computer was programmed to capture and report the Stress force at 5% Strain. This is a common value and common methodology utilized in the web industry.

When a web is constructed to the concepts described herein, such as having a repeating pattern of predominant high stress lanes aligned normal to the stretch force axis and interconnected to the stretch force axis by connecting members, one will have a form fitting flexible web that will move with the changes of the anatomy. The web will remain in intimate contact with the body avoiding any displacement, wrinkling or bunching, thus maintaining a constant comfort factor.

While the webs described herein are readily useful as a liner in women's panties, disposable panties, or disposable absorbent articles inserted into the panties, one skilled in the art will recognize that it may be useful in other devices such as feminine napkins, diapers, incontinence devices, wound dressings, bandages, bed pads, bed linens, surgical drapes or many other devices where the body is contacted by the web. It is also noteworthy that when the webs described herein are applied to liners, pads, napkins, bandages or other such devices, it is desirable they be affixed to such absorbent articles, which are themselves also made flexible by comprising any combination of a known variety of flexible absorbent core and flexible barrier layer constructions.

A number of embodiments of the invention have been described. Nevertheless, it will be understood that various modifications may be made without departing from the spirit and scope of the invention. Accordingly, other embodiments are within the scope of the following claims.

## Claims

1. A web comprising:
a repeating pattern of elongated high stress lanes aligned substantially normal to a stretch force axis;
the pattern of high stress lanes being defined by a plurality of depressions which protrude away from an uppermost contacting plane of the web, the depressions having substantially vertical sidewalls;
the high stress lanes having a width defined by a distance between the substantially vertical sidewalls of the depressions on either side of the lane;
the repeating pattern of lanes having a separation distance between the lanes; and
the depressions being interrupted by connecting members extending between adjacent high stress lanes.

2. The web of claim 1 wherein the connecting members are in a lower plane than an uppermost plane of the high stress lanes.

3. The web of claim 1 wherein the connecting members are essentially coplanar with an uppermost plane of the high stress lanes.

4. The web of claim 1 or 2 wherein the high stress lanes exhibit twisted thread lines.

5. A flexible form fitting web comprising:
a plurality of elongated depressions having substantially vertical sidewalls along a length of the depressions and connecting members at ends of the depressions;
a plurality of elongated high stress lanes between the depressions, the high stress lanes extending in a machine direction and being connected to one another by the connecting members and being separated from one another by the depressions; and
the high stress lanes and the connecting members each having an upper surface substantially in the same plane.

6. The web of any one of the preceding claims wherein the 5% Stress value for a stress force axis is no more than 30% of the 5% Stress value in a high stress lane axis.

7. The web of any one of the preceding claims having a lane width of at least 10µm to at most 5,000µm.

8. The web of claim 7 wherein the lane width is at least 300µm to at most 1,500µm.

9. The web of any one of the preceding claims wherein the lanes repeat with a separation distance of at least 2% of the lane width to at most 400% of the lane width.

10. The web of claim 9 wherein the lanes repeat with the separation distance from 100% of the lane width to 200% of the lane width.

11. The web of any one of the preceding claims wherein the connecting members interrupt the depressions once every 1,250µm to once every 12,500µm.

12. The web of any one of the preceding claims made from a blend comprising no elastomeric polymers.

13. The web of any one of the preceding claims wherein patterned zones of apertured and unapertured depressions coexist.

14. The web of any one of the preceding claims having a depression depth of at least 25% of the lane width to at most 500% of the lane width.

15. The web of claim 14 wherein the depression depth is from 100% of the lane width to 300% of the lane width.

16. The web of any one of the preceding claims wherein the depressions have substantially pointed ends for unhinging during bending.

17. The web of claim 15 where the depressions are apertures.

18. An article for the absorption of bodily fluids comprising:
a flexible absorbent core, a flexible barrier layer, and a flexible body contacting layer with a repeating pattern of predominant high stress lanes aligned normal to a stretch force axis; the pattern of high stress lanes being defined by depressions which protrude away from the uppermost contacting plane of the web; and
a lane width being effectively defined by a distance between a substantially vertical component of the depressions on both sides of the lane; the repeating pattern of lanes being defined by a separation distance between the lanes; the depressions being interrupted by connecting members; the connecting members transmitting the stretch force to the high stress lanes by being affixed to sides of the high stress lanes.
